# EUROPEAN PATENT SPECIFICATION

(11) **EP 0 756 519 B1**
(45) Date of publication and mention of the grant of the patent: **05.12.2001**
(21) Application number: 96903842.1
(22) Date of filing: 16.02.1996
(51) Int. Cl.: B01J 27/198, C07C 51/215

(54) **PROCESS FOR PREPARING AN OXIDATION CATALYST AND USE THEREOF**
VERFAHREN ZUR HERSTELLUNG EINES OXYDATIONS KATALISATORS UND VERWENDUNG DERSELBEN
PROCEDE DE PREPARATION D'UN CATALYSEUR D'OXYDATION ET SON UTILISATION

(30) Priority: 17.02.1995 WO PCT/BE95/00016
(43) Date of publication of application: 05.02.1997
(73) Proprietor: PANTOCHIM S.A., 7181 Feluy (BE)
(72) Inventor: NSUNDA KINKELA,Veron, B-5000 Namur (BE)
(74) Representative: Schmitz, Yvon
(86) International application number: BE9600015
(87) International publication number: WO9625230

(56) References cited:
- EP-A- 0 471 853
- EP-A- 0 518 548
- US-A- 4 336 198

## Description

### FIELD OF THE INVENTION

This invention relates to a method of making a supported vanadium-phosphorus mixed oxide catalyst for the production of maleic anhydride in a fixed-bed reactor. More particularly, the present invention provides selective, active, low cost catalysts suitable for commercial production of maleic anhydride by oxidation of aliphatic hydrocarbons in the vapor phase.

### BACKGROUND OF THE INVENTION

Maleic anhydride is a substantial commercial product made throughout the world for over fifty years. It is used alone or in combination with other materials mostly as a precursor for other products, including resins, pharmaceuticals, and food additives.

Two types of commercial reactors are used for the manufacture of maleic anhydride : fixed-bed )multi-tubular and fluid-bed reactors. The greatest part of maleic anhydride is produced in fixed-bed multi-tubular reactors.

The catalyst used in all of the commercial reactors is a vanadium phosphorus oxide type. About a thousand articles and patents have been published related to the vanadium phosphorus oxide catalysts, since Bergman et al., U.S. Patent N° 3,293,268 taught the process of oxidizing saturated aliphatic hydrocarbons to produce maleic anhydride using such catalysts, often referred to as mixed oxides of vanadium and phosphorus. Bulk analysis of the active, mature catalyst shows the catalyst to be generally vanadyl pyrophosphate. However, as yet there are many factors not clearly understood that are important to the making of active, mature catalysts giving commercially acceptable productivities, yields, and lives.

Numerous methods of making the vanadyl phosphate catalysts with and without promoters are disclosed and taught in the prior art. Generally, such catalysts are made by contacting suitable vanadium compounds and phosphorus compounds under conditions which result in most of the vanadium being in the +4 valence and reacted with the phosphorus to form a catalyst precursor comprising hydrated vanadyl hydrogen phosphate. The catalyst precursor is subsequently recovered by techniques well known in the art, such as decanting, filtering, and centrifuging, and treated physically and thermally by several conventional practices to form "calcined" mature catalysts.

In general, two main procedures for the preparation of the catalyst precursor are followed : 1) the reduction in the aqueous phase of a vanadium compound followed by the addition of a phosphorus compound and the separation of the solid either by water evaporation or by crystallization, and 2) the partial or total reduction in the organic phase of the vanadium compound with an organic or inorganic reducing agent, followed by the addition of a phosphorus compound and separation of the solid.

In addition to the presence of vanadium and phosphorus, these catalysts can also contain modifying components such as molybdenum, iron, zinc, titanium, uranium, and niobium as well as diluents such as silica and alumina.

Specific samples of suitable catalyst precursors are described in several patents and publications (U.S. Patent Nos. 4,632,915 ; 4,562,268 ; 4,333,853 ; 4,315,864 ; 4,132,670 ; 4,064,070 ; J. W. Johnson et al., J. Am. Chem. Soc., **106**, 8183 (1984) ; F. Cavani et al., Applied Catal., **9**, 191 (1984) ; R. S. Horowitz et al., Applied Catal., **38**, 193 (1988) ; R. S. K. Bej et al., Applied Catal., **83**, 149 (1992) ; R. Sant et al , J. Catal., **143**, 215 (1993). It is understood that the references are not to be construed as limiting, but are for purposes of illustration and guidance in the practice of the instant invention.

The methods used for the calcination and activation of the catalyst precursor may be divided into two categories : 1) calcination performed in equipment )other than the reactor (*ex-situ* calcination), and 2) calcination in the reactor tubes, usually under mild operating conditions in presence of hydrocarbon and air (*in-situ* calcination).

U.S. Patent N° 5,137,268 teaches a process for conversion of vanadium-phosphorus catalyst precursors to active catalysts by subjecting the catalyst precursors structures, which are beforehand placed in a box oven, to elevated temperatures in three stages : a) an initial heat-up stage in an atmosphere of air, steam and nitrogen, (b) a rapid heat-up stage at a programmed heat-up rate in an air/steam atmosphere and (c) a maintenance-finishing stage, using consecutively an oxygen-containing and a non-oxidizing atmosphere.

U.S. Patent N° 4,562,268 relates to a process for the production of maleic anhydride by oxidation of aliphatic hydrocarbons in the vapor phase using phosphorus-vanadium mixed oxide catalysts. The patent discloses two basic modes of calcination : (1) air calcination and (2) nitrogen/steam calcination. In the air calcination the catalyst precursors are subjected to heating in air, as in one embodiment, to 400°C over a two hour period, then held at this temperature for six hours. In the nitrogen/steam calcination, the catalyst precursors are placed in a tube furnace and first calcined in air at a temperature in the range from 325°C to 350°C for six hours, followed by calcination in nitrogen and steam at a temperature in the range from 250°C to 600°C for from two to ten hours.

U.S. Patent N° 4,392,986 discloses a process for preparing vanadium-phosphorus catalyst by reaction of vanadium compound and phosphorus compound in isobutanol followed by water washing of the precursor. The precursor, after drying at 120°C, is activated in the reactor oxidizing butane in air to maleic anhydride, typifying the *in-situ* calcination type.

U.S. Patent N° 4,317,777 teaches the production of maleic anhydride using vanadium-phosphorus catalysts which were calcined *in-situ* by heating to 385°C at a rate of 9°C/minute, whilst a 1.5% v/v n-butane/air mixture flowed through the bed at a GHSV of 1000 hr⁻¹.

Numerous patents disclose improved catalyst systems and methods and processes for preparation of catalysts containing the mixed oxides of phosphorus and vanadium which exhibit high weight to weight productivities of maleic anhydride.

W.O. Patent Application N° 94/13,401 describes an active, phosphorus vanadium oxide catalyst for the conversion to maleic anhydride of a non-aromatic hydrocarbon having at least four carbon atoms in a straight chain, said catalyst comprising a shaped body having a volume of at least about 0.02 cc and containing a promoter in such a proportion as to enable the catalyst to have developed surface area of at least about 28 m²/g and to exhibit a weight/area productivity of at least about 3.5 mg maleic anhydride/m²-hr and/or a weight/weight productivity of at least about 100 g maleic anhydride/kg.cat.-hr. when contacted with a gas flow volume to catalyst weight ratio of 2180 cc/g-min. under a pressure of 1.055 x 10²-kPa-G, and at a temperature sufficient to maintain a hydrocarbon conversion of 85 mole percent.

U.S. Patent N° 5,011,945 discloses a recycle process for continuous oxidation of n-butane to maleic anhydride wherein product yields are high and catalyst productivity is within the range of from about 0.12 to about 0.18 measured in pounds of maleic anhydride produced per pound of catalyst per hour. Use of co-metal modified catalyst of vanadium phosphorus oxygen, wherein the co-metal is molybdenum, as well as contiuous addition of water vapor with an alkyl ester of orthophosphoric acid are required for increased yield, selectivity and catalyst productivity.

U.S. Patent 4,562,268 (and its family patents U.S. 4,567,158 ; U.S. 4,560,674 ; E.P. 0,151,912) relates to a process for the production of maleic anhydride from non-aromatic hydrocarbons in the presence of a phosphorus vanadium mixed oxide oxidation catalyst wherein the catalyst exhibits, after being formed into desired structures and calcined at a temperature from about 250°C to about 600°C, a single pass weight/weight productivity of at least 70 grams of maleic anhydride per kilogram of catalyst per hour based upon a performance test conducted at a concentration of 1.5 mole percent hydrocarbon in molecular oxygen-containing gas, a space velocity of 1450 hr⁻¹ under a pressure of 1.055*10² kPa-G, and a temperature sufficient to maintain the hydrocarbon conversion within the range of 70 mole percent to 90 mole percent.

U.S. Patent N° 4,713,464 provides a process for the preparation of a catalyst containing a vanadium/phosphorus complex oxide which is distinguished, as compared to known catalysts, by easier and more rapid conditionning and especially by higher activity, selectivity and productivity. The catalyst according to the invention is characterized in that the volume of the pores with a radius of 100 to 1,000 angstroms is at 30% of the total pore volume of the pores which have a radius of less than 10,000 angstroms and exhibits a hourly maleic anhydride production rate of 150 grams per kg of catalyst.

U.S. Patent N° 4,092,269 discloses a method for preparing an improved phosphorus-vanadium-oxygen catalyst useful in the manufacture of maleic anhydride by the oxidation of saturated hydrocarbons in higher yields, wherein a phosphorus compound and a vanadium compound are brought together under conditions to provide a catalyst precursor having a phosphorus to vanadium ratio between about 0.5:1 and 2:1, and calcining the precursor at a temperature between 300° and 600°C, the improvement which comprises adding to the precursor a sufficient amount of a pore modification agent selected from the group consisting of polymeric materials, monosaccharides, hydrogenated vegetable oils and mixture thereof, more preferably )methylcellulose, to provide a catalyst wherein the volume of pores having diameters between about 0.8 micron and about 10 microns is greater than 0.02 cc/g.

The potential methods of forming the vanadium phosphorus precursors into shapes suitable for the use in the available reactors are described and are well known in the art. In the multi-tubular fixed-bed reactor, usually fabricated with thousands of small diameter (e.g. 21 mm) metal tubes immersed in molten salt heat transfer system, the main factors defining the shape are diameter of the tubes, pressure drop, heat transfer, and availability of the catalyst surface to the reactants. In the fluidized bed reactor fluidizing properties and attrition resistance are major concerns. Extrudates, tablets, pellets, fine particles, in the form of many shapes have been reported. These forms are generally made from undiluted, unsupported active catalyst or catalyst precursor and may be referred to as full or 100% catalyst.

U.S. Patent N° 5,168,090 has 16 sheets of drawing on shaped oxidation catalyst structures comprised of mixed oxides of vanadium and phosphorus. Its claims include cylinders, cubes, cones, truncated -cones, truncated pyramids, spheres, and prisms having void )spaces of grooves, holes, and dimples both angular and rounded shapes, all obviously for use in fixed-bed reactors.

U.S. Patent N° 4,283,307 claims a catalyst structure comprising a cylinder having a bore therethrough consisting essentially of catalytic material, which is comprised of phosphorus-vanadium-oxygen complex.

U.S.Patent N° 4,769,477 teaches an attrition resistant vanadium-phosphorus oxide catalyst for use in a fluidized bed utilizing an aqueous silicic acid solution, the relative amounts of the particles and silicic acid chosen so that the weight of SiO₂ is about 3-15% of the total weight of the vanadium-phosphorus oxide particles and the SiO₂, the silicic acid containing silica having a particle size no greater than 5 nm.

E.P. Application N° 107,274 relates to a process for the preparation of attrition resistant, microspheroidal fluid-bed catalysts comprising the mixed oxides of vanadium and phosphorus in which a vanadium phosphorus mixed oxide catalyst precursor is densified, comminuted, formed into fluidizable particles and calcined under fluidizable-type conditions. It is claimed that the process is characterized in that the fluidizable particles are formed by introducing the catalyst precursor into water to form an aqueous slurry and spray-drying said slurry to form microspheroidal catalyst particles.

U.S. Patent N° 4,351,773 provides a process for the preparation of fluid-bed oxidation catalysts containing the mixed oxides of vanadium and phosphorus, including the steps of preparing the catalyst precursor, comminuting the precursor, introducing the precursor into water to form an aqueous slurry and spray-drying the slurry to form microspheroidal catalyst particles having a particle size range between 20 to about 300 micron.

G.B. Patent N° 1,285,075 discloses a process in which unsaturated hydrocarbons containing four or more carbon atoms are oxidized in the presence of a fluidized bed catalyst containing a vanadium phosphorus oxide system which have been prepared by spray-drying a mixture of vanadium compound, a phosphorus compound and an aqueous silica sol to obtain solid particles having a mean diameter ranging from 60 to 200 microns, and calcining the said solid particles.

While many patents allude to active catalyst material deposited on carrier as a possible way of making a vanadium phosphorus mixed oxide catalyst, very few have actual examples and claims including the use of inert solids as supports of active catalysts.

U.S. Patent N° 4,632,915 gives no examples and no specific claims on the use of supported catalyst even though it summarizes a well known reference to the possible use of supported catalyst.

E.P. Application N° 623,576 claims a process for the selective oxidation of hydrocarbons, which may or may not be saturated, in the presence of a catalyst comprising a support based on one or more metal oxides, and vanadium-phosphorus oxide in amount of from 0.01 to 45%, based on the weight of the catalyst and calculated as vanadyl pyrophosphate [(VO)₂P₂O₇], which process comprises two steps, one of oxidation and the other of reduction phase, wherein a hydrocarbon is contacted with said catalyst in the reduction phase and in oxidized or non-oxidized form is adsorbed onto the catalyst, whereafter the thus loaded catalyst is brought into the oxidation phase, the desired product being formed in the presence of gaseous oxygen and subsequently separated. In a preferred embodiment of )the invention, the catalyst is prepared by the method of deposition-precipitation from homogeneous solution on suspended supports. The catalyst preparation process comprises the following steps : a) electrochemical reduction of vanadium (V) compound in acid solutions of a pH < 2 to vanadium having an initial average valence of 2.9, b) adjustment of phosphorus to vanadium ratio of 1.1:1, c) increase of pH, from 2 at the start to 7.0 at the end, in a suspension of titanium oxide (Degussa P25) with a load of 8.8 percent by weight, based on (VO)₂P₂O₇, and with exclusion of oxidizing components. The catalyst sieve fraction 0.09-0.015 mm, which is generally used in fluid-bed reactor, is loaded with butane (10 to 20% in argon, space velocity of 2500 per hour) in a fixed-bed reactor for 5 minutes and subsequently, in a separate step, after flushing with argon at a space velocity of 5000 per hour, it is reoxidized for 2 minutes with a varying amount of oxygen (20 to 4% oxygen in argon, space velocity of 2500 per hour). The selectivity to maleic anhydride is relatively low as compared to standard performant catalysts. It varies at the maximum amount of product stream between 33 and 40%, for oxygen concentration varying from 20 to 2%.

The widespread allusions to the use of a supported catalyst may have arisen from the fact that coated supported vanadium catalysts have been used commercially for many years for the oxidation of benzene to maleic anhydride, U.S. Patent N° 3,106,569. Prior to the first commercial production of maleic anhydride from butane in 1974, the 1969 U.S. Patent N° 3,178,063 claimed the use of up to 90% carrier in a process using a vanadium phosphorus mixed oxide catalyst, but only for oxidation of olefinic unsaturated aliphatic hydrocarbons (e.g. butene used commercially at the time) to maleic anhydride, not for saturated aliphatic hydrocarbons.

G.B. Patent N° 1,478,523 provides a process for the manufacture of maleic anhydride and/or maleic acid by oxidation of unsaturated aliphatic hydrocarbons of not less than 4 carbon atoms using molecular oxygen or a molecular oxygen-containing gas at a temperature from 300 to 500°C in the presence of supported catalysts which, unlike the catalysts used for the oxidation of saturated aliphatic hydrocarbons, contain a vanadium compound in a valence state of +5. The supported catalysts are produced by mixing an aqueous solution or suspension of V₂O₅ and of P₂O₅ with finely divided anatase (TiO₂) to give an aqueous suspension which is sprayed onto the carrier which is heated, for example to from 120 to 450°C. The amounts of the catalytically active material in the supported catalysts is from 50 to 1,500% by weight, based on the carrier.

U.S. Patent N° 4,312,787 and its division, U.S. Patent N° 4,336,198, are among the few patents giving examples and claiming coating of catalytically active vanadium phosphorus mixed oxides containing essentially vanadyl pyrophosphate (in which the average vanadium valence is close to +4) on inert supports for the preparation of maleic anhydride from n-butane. The basic method for preparing these catalysts is to partially wet the supports material with a liquid. These partially wet supports are then contacted with a powder of the catalytically active material with or without support material and the mixture is gently agitated until the catalyst is formed. However, the catalyst art taught and claimed is restricted to a coating amount greater than 50% to about 80% by weight of the combined support and active oxide. The claim is made therein that the performance is inferior when the catalytic oxide material is present in an amount less than 50% by weight.

U.S. Patent N° 4,071,539 discloses a complex vanadium phosphorus oxide catalyst modified with copper and tellurium oxides and suggests coating onto carriers. The amount of coating to achieve performance comparable with non-coated (100%) catalysts is 85% to over 90% of the combined weight of the coating and carrier.

The above patents argue that the percentage of weight of the combined coating and carrier is a significant variable. The results of the instant invention show that another, maybe the more significant variable is the thickness of the coating and the weight of the active catalyst per unit bulk volume of the combined coating and carrier. It is obvious that for the claims of 50% through 90% weight to weight ratios of the active catalyst to combined coating and carrier, the weight of the coating may approach the 100% catalyst if the carrier is light in weight. Also, for carriers that differ greatly in density the weight percents of the coating will vary whereas the amount of the active catalyst may be the same in a given oxidation reactor, Thus, in a coated catalyst having 50% active coating and a bulk density of 1.0 g/cm³, the active coating is present in the ratio of 0.5 g/cm³ of bulk volume. In a coated catalyst having equally 50% active material, but a bulk density of 0.6 g/cm³, the active coating is present in the ratio of 0.3 g/cm³ of bulk volume. This is a significant catalytic difference, since the reactors are limited by the volume of the catalyst space (volume of the tubes in a multi-tubular reactors). The differences in the weight of the coated catalysts may be the result of the carriers being made of lower density material, e.g. a porous diatomaceous earth, or the result of the carrier )having a higher void volume, e.g. thin wall Pall or Raschig rings, or a combination of both factors.

It appears from the above-described prior art that the use of water during coating may limit the performance of supported vanadyl pyrophosphate catalysts for the manufacture in fixed-bed reactor of maleic anhydride from saturated aliphatic hydrocarbons. It is an advantage of the present invention that a coated vanadyl pyrophosphate based catalyst which contains low amounts of catalytically active material and exhibits high activity, selectivity and/or productivity is obtained.

### SUMMARY OF THE INVENTION

Thus, it is an object of the instant invention to provide a new, improved process for the preparation of active efficient coated catalysts having not only thin layers of active catalytic material, 0.1 mm to 0.8 mm in thickness, and with low percent by weight active coating, 3% to 40%, but also having low weight amounts of active coating per unit bulk volume of the combined coating and carrier, namely 0.02 to 0.40 grams and preferably 0.03 to 0.35 grams of active material per cubic centimeter bulk volume, the said active coating being characterized after calcination by a pore radius average between 270 and 2500 angstroms and a percent porosity of at least 30 %, as measured with a mercury porosimeter.

According to the present invention, this object is attained by a process for the preparation of an oxidation catalyst containing an active catalytic material coated in a thin layer on the surface of a solid support, said active catalytic material containing vanadyl pyrophosphate, having an average vanadium valence from 3.8 to 4.5 and a phosphorus to vanadium atomic ratio from 0.8 to 1.5, being present in an amount from 0.02 grams to 0.40 grams per cubic centimeter bulk volume of the combined support and active catalytic material, and having a thickness of from about 0.1 mm to 0.8 mm, which comprises the following steps:
a) providing an active catalyst precursor containing vanadyl hydrogen phosphate hemihydrate;
b) mixing said active catalyst precursor with an organic liquid;
c) coating said mixture onto a solid support;
d) calcining the above mixture coated on the solid support in the presence of a gas mixture of 50/50 air and steam to obtain said active catalytic material, and thus to produce a catalyst with an active catalytic material having a pore radius average of 270 to 2500 angstroms and a percent porosity of at least 30 % and exhibiting a weight/weight productivity to maleic anhydride of at least 200g maleic anhydride/hour per kg active catalytic material based on a performance test conducted in a fixed-bed reactor at a concentration of 1.5 mole percent hydrocarbon in molecular oxygen containing gas, a space velocity of 1400 hr⁻¹ under a pressure of 1 atmosphere absolute, and a temperature sufficient to maintain the hydrocarbon conversion of 60 mole % to 85 mole %.

Another object of this invention is to provide an improved process for the preparation of coated oxidation catalysts containing mixed oxides of vanadium and phosphorus which are suitable for the production of maleic anhydride, with a weight/weight productivity, in a fixed-bed reactor, of at least 200 g maleic anhydride per hour per kg of active catalytic material, via the partial oxidation of from C₄ to C₁₀ aliphatic hydrocarbons in the vapor phase with molecular oxygen or a molecular oxygen containing gas. The use of such coated catalysts results in a lower manufacturing cost, a more efficient use of the active material, higher heat transfer, and lower temperature profiles.

This and other objects, facets, and advantages of the instant invention will become apparent to those skilled in the art from the accompanying description and claims.

### DESCRIPTION OF THE PREFERRED EMBODIMENTS

The process of this invention presents a new dimension of economy and efficiency in providing vanadyl pyrophosphate catalysts using a minimum but effective amount of the active mixed oxides useful for the production of maleic anhydride in fixed-bed reactor by the partial oxidation of one or more C₄ to C₁₀ aliphatic hydrocarbons in the vapor phase with molecular oxygen or a molecular oxygen containing gas. This small amount of active material is coated as a thin layer on the surface of carriers. In this context the word thin means a coating with a thickness ranging from 0.1 mm to 0.8 mm. The coated catalysts prepared according to the instant invention, using only a reduced amount of active material, remarkably give conversions and selectivities of maleic anhydride equal to or better than those of catalysts used in conventional commercial operation, as well as extremely high productivity based on the active catalytic material.

The following are the main steps in a preferred embodiment:
a) providing an active catalyst precursor containing vanadyl hydrogen phosphate hemihydrate with one or more modifying components;
b) mixing said active catalyst precursor with an organic liquid and optionally a binder;
c) coating said mixture onto a solid support of selected material, shape and dimensions;
d) calcining the catalyst precursor mixture thus coated on the solid support either in the reactor *(in-situ*) after loading or external *(ex-situ)* prior to loading in the reactor, so that the resulting calcined catalyst has an average valence from 3.8 to 4.5, preferably from 3.9 to 4.3 and a phosphorus to vanadium atomic ratio from 0.8 to 1.5, preferably from 0.9 to 1.2 and is
characterized by a pore radius average of 270 to 2500, preferably from 350 to 2200 angstroms and a percent porosity of at least 30 %, as measured by mercury porosimeter.

As heretofore stated the catalyst precursor may be prepared (step a) by several different procedures (see Background of the Invention). A particularly suitable method of making the precursor is illustrated in Example 1. The active catalyst material for coating is a catalyst precursor powder or, more preferably, a wet cake of a catalyst precursor which may be diluted and intermixed with an inert diluent. Moderate dilution of the active catalyst or precursor by admixing an inert diluent to modifiy the catalyst activity or to decompose, in order to increase porosity, is well known.

The liquid (step b) in which the active catalyst precursor is slurried is an organic compound such as, for example, an alcohol, an ether, a ketone, an amine, a carboxylic acid derivative, a hydrocarbon or a mixture thereof. The organic liquid in general supplies a vehicle to allow deposition of the precursor onto the solid inert support.

The use of a binder (step b) is optional. However, the use of an effective binder ensures that the active material adheres to the support during the calcination phase and operation at high temperatures. Made without a binder, the catalyst, while its use is more advantageous than the full (100%) counterpart, may dust and have disadvantages when compared to the catalyst made with a binder.

Several binder types are satisfactory, for example but non-limiting, polyvinyl alcohols of varying molecular weights, polyols, sugars and their derivatives, cellulose and its derivatives, oxalic acid and its derivatives, ammonium salts of organic and inorganic acids, stearic acid and its corresponding salts, and ammonium phosphate. Particularly effective binders are polyethyleneglycols having a (non-limiting) range of molecular weight of from about 3,000 to about 45,000, and glucose.

The carriers (step c) on which the active catalyst is coated may be composed of a number of different materials well known in the general catalytic field. They may consist of, but are not limited to silica, alumina, silica-alumina, natural and synthetic silicates containing aluminum or magnesium or both, magnesia, pumice, kieselgur, niobium oxide, silicon carbide, stainless steel, titania, titania-silica, ceramic materials, and zirconia. Ceramic materials, silicon carbide, and silica-alumina are preferred.

The carriers can have various shapes. They may be of regular geometric shapes including full spherical, or spheroid forms and full cylinders with holes and indentations on the outer surfaces. They may be cored cylinders, Berl saddles, and fragmented particles. In fixed-bed multi-tubular reactors, cored cylinders and Berl saddles are especially effective in presenting the catalytic material to the reacting gas stream and in providing low pressure drop. Cylinders and spheres are preferred.

The coating of the carrier can effectively be accomplished by procedures described in Examples 2, 3, 4, 5, 6 and 7, Surprisingly, it has been discovered that when an organic liquid, instead of water, is used, in order to deposit the active catalytic material on the carrier (Step c), improved coated catalysts are obtained which are characterized, after calcination (Step d) by well-defined pore radius average and percent porosity values, ranging from 270 to 2500 angstrom and from 30 to 70%, respectively, and exhibiting high productivity at lower reaction temperature in a fixed-bed reactor. Alcohols and hydrocarbons have been found to be particularly effective. Isobutyl alcohol and xylene are preferred.

It also has been discovered that, contrary to certain claims in the prior art (see for instance U.S. 4,312,787 ; U.S. 4,071,539), when, according to the present invention, an organic solvent, instead of water, is used to carry out coating of active material on the carrier (see Example 6 ), the amount of said active material required to achieve yields of maleic anhydride comparable to yields achieved with non-coated (100%) catalysts, is comprised in the range of 3 to 40%. When the active material is present in an amount higher than 30% of the combined weight of the catalytic vanadium-phosphorus mixed oxide material and support, no significant increase of maleic anhydride yields are observed (see Table 1). This improved coated oxidation catalyst presents the advantage of filling partially the reactor tubes with a low cost carrier and of using more efficiently the active material.

According to the present invention, the use of an organic solvent, more particularly an alcohol or an hydrocarbon, for the preparation of the slurry containing the catalyst precursor to be deposited onto the surface of the carrier, combined with the coating procedure, and followed by the calcination step (see below), leads to the formation of an improved coated vanadyl pyrophosphate catalyst which
1) is characterized by an average vanadium valence of 3.8 to 4.5, preferably of 3.9 to 4.3, a phosphorus to vanadium atomic ratio of 0.8 to 1.5, preferably of 0.9 to 1.2, a pore radius average of 270 to 2500 and preferably of 350 to 2200 angstroms, and a percent porosity of at least 30%, and
2) exhibits a weight/weight productivity to maleic anhydride of at least 200 g maleic anhydride/hour per kg of active material when tested in a fixed-bed tubular reactor at a concentration of 1.5 mole percent hydrocarbon in molecular oxygen-containing gas, a space velocity of 1400 hr⁻¹ under a pressure of 1 atmosphere absolute, and a temperature sufficient to maintain the hydrocarbon conversion of 60 mole % to 85 mole % as described hereinafter.

In a simple coating procedure, the carrier and a slurry of catalytic material are mixed in a porcelain vessel and stirred gently with a porcelain spatula, while heating on a steam bath until the mixture is dry in appearance. In this method care should be taken that the stirring is not too vigorous to dislodge the coating during a sticky, thick stage.

An effective method, which is the preferred procedure of the present invention, involves atomizing the slurry of active catalytic material with or without a binder, with an air stream, and directing the fine droplets onto a carrier in a rotating heated vessel. The active catalytic material used for the preparation of the slurry may be a dried precursor powder or a wet cake precursor material. Organic liquids are used for the preparation of the slurry. As illustrated in Examples 5, 6 and 7, superior catalysts are obtained when isobutyl alcohol or xylene is used.

Another procedure, described in Example 4 uses commercial coating machine, namely a Hüttlin turbojet driving unit (model HKC-5-TJ) equipped with spray nozzles and heater air blowing capabilities.

The coated catalyst may be calcined in-situ but preferably the coated catalyst is calcined *ex-situ*. A particularly useful procedure entails charging the catalyst to a 50 mm diameter borosilicate tube and placed in a vertical Lindberg oven. Before starting the heating process, a 50/50 mixture air/nitrogen is passed over the catalyst. When the temperature reaches 150°C, over 4 hours, the atmosphere in the tube is changed to 50/50 mixture of air and steam. The temperature is then raised to 420°C, over 10 hours, and maintained for 4 hours. The catalyst is then cooled under nitrogen, being ready for performance testing.

For the purpose of comparing the performance efficiencies of catalysts made pursuant with the instant invention with comparative technologies, the active catalyst forms may be tested using a variety of reactor types which are well known in the art. In the instant invention, the comparison is made by reacting a hydrocarbon, usually n-butane, as an admixture with air, on a sample of catalyst in a single tube reactor. A volume of 60 ml of catalyst is charged to a 21 mm diameter stainless steel reactor to an approximate depth of 180 mm and the reactor submerged in a liquid mixed salt bath. The salt bath used is a mixture of potassium nitrate, sodium nitrate and sodium nitrite, the eutectic being a common commercially used heat transfer medium. The feed concentration of n-butane in air employed is 1.5% by volume, with the gas volume hourly space velocity (GHSV) of 1400 volumes of gas per volume of catalyst at standard conditions. The tests are carried out at from about 300°C to 550°C and more preferably from about 350°C to 460°C salt bath temperature.

The performance variables normally measured and calculated include conversion (usually one-pass conversion) of the feed hydrocarbon, yield of maleic anhydride based on the feed hydrocarbon and selectivity to maleic anhydride. The percent conversion is expressed as the moles of hydrocarbon reacted per 100 moles of butane fed. The percent yield of maleic anhydride is expressed as the moles of maleic anhydride produced from 100 moles of butane fed to the reactor. The percent selectivity to maleic anhydride is expressed as the moles of maleic anhydride formed from 100 moles of butane reacted.

A significant way of characterizing the catalysts of the instant invention, with respect to the full, 100% catalyst of comparative technologies, is the productivity of maleic anhydride based on the amount of active catalytic material. The productivity measured on the active material basis may be expressed as weight of maleic anhydride per weight of active material and time. The units used herein are grams of maleic anhydride per kilogram of active material per hour. The so-called Active Material Productivity (A.M.P.) signifies the worth of the catalysts in the instant invention, with A.M.P.s over ten-fold the current 100% catalyst technology.

### Example 1

This example illustrates a suitable procedure for preparation of the catalyst precursor.

A 10 litres, four-neck, round-bottom flask, fitted with a mechanical stirrer with a 15 cm teflon paddle, a thermometer, a heating mantle and a reflux condenser, is charged with 6480 ml (5156 g) of isobutyl alcohol and 720 ml (750 g) of benzyl alcohol. Stirring is started (about 350 r.p.m.) and 670 g (3.7 moles) of vanadium pentoxide (V₂O₅) are added. The mixture is heated to reflux temperature, about 107°C, and maintained at reflux for three hours. After the initial reflux period, the stirred mixture is cooled down to about 20°C below the reflux temperature and 816 g (8.3 moles) of 106% phosphoric acid are added. The resultant mixture is again heated to reflux and maintained at reflux for 16 hours. This mixture is cooled to about 50°C and suction filtered to yield a bright blue cake. The cake may be used as such for the coating operation or, if desired, transferred to four open 2-litre dish trays and dried in a forced air oven at 150°C for 10 hours to give approximately 1300 g of a grey-blue catalyst precursor powder.

### Example 2 (Comparative)

This example is outside from the scope of the invention and describes a coating process using water as a liquid for the preparation of the slurry containing binder.

Approximately 200 ml of solid supports are introduced in a cylindrical container (diameter 100 mm and height 200 mm) containing 60 ml of a 5% (by weight) aqueous solution of polyvinyl alcohol and caused to rotate (about 50 r.p.m.). When the supports have been fully soaked in this solution, approximately 25 g of precursor powder are introduced in the container. After one or two minutes of contact, rotation is stopped and the coated solid supports are placed in a crystallizer and then dried for 15 minutes at 150°C in a furnace with a circulation of air. The coated supports are then cooled to room temperature and replaced in the rotating cylinder, containing 20 ml of aqueous binder solution. Coating is continued after introduction of 10 g of precursor powder, then the coated supports are dried in the same way as indicated above. This operation is repeated until the desired quantity of precursor is fixed on the solid support. After calcination in a vertical tubular furnace, the catalysts are performance tested in the test reactor described herein. The carrier, binder and catalyst characteristics are detailed in Table 1 as referred to catalysts 2a, 2b, 2c, 2d, 2e, and 2f.

### Example 3 (Comparative)

This example illustrates another coating procedure using also water as a liquid for the preparation of the slurry containing catalytic material.

10 g of polyvinyl alcohol (or of another binder) are slowly poured into a 500 ml flask containing 190 ml of water maintained under agitation. After complete solubilization of the binder, 50 to 100 g of precursor powder are added in small portions to the solution, maintaining the agitation. The suspension is then poured into the reservoir of a paint gun and sprayed on the surface of solid supports of various materials, shapes, and dimensions that are placed in a rotating (20 r.p.m.) cylindrical container and heated to about 100°C by means of a stream of hot air. The quantity of the solid supports introduced into the cylindrical container depends on the amount of active material to be deposited. The coated catalysts obtained in such a way are then calcined in a vertical tubular furnace as describe herein. The carrier, binder and catalyst characteristics are detailed in Table 1 as referred to catalysts 3a and 3b.

### Example 4 (Comparative)

This example, which is also outside from the scope of the present invention, illustrates a coating process, using an aqueous solution of the catalyst precursor and a commercial coating machine to fix the catalyst precursor powder on solid supports of various forms and dimensions.

About 3000 g of carriers are placed in a Hüttlin turbojet driving unit (model HKC-5-TJ) equipped with 3-component spray nozzles with diameters of 1.2 mm. The carriers are gently moved horizontally with blowing air (600 cubic metre/hour) preheated at 68°C. Then, a slurry containing 600 g of catalyst precursor powder and 2500 g of a 2.5% (by weight) aqueous solution of polyvinyl alcohol is sprayed on the moving carriers at a rate of about 30 to 40 g of slurry per minute. After completion of the spraying process, about 85% of the precursor powder is coated onto the carriers. After calcination in a vertical tubular furnace as described herein, 60 ml of catalyst sample is tested in the single tube reactor. The carrier, binder, catalyst characteristics are detailed in Table 1 as referred to catalysts 4a, 4b, 4c, 4d, and 4e.

### Example 5

This example illustrates the preferred coating procedure of this invention where, isobutyl alcohol and a precursor powder are used to prepare the slurry.
Approximately 200 ml of solid inert support are placed in a rotating (20 r.p.m.) cylindrical container and heated at about 50°C by means of a stream of hot air. Depending on the quantity of active material to be deposited on the surface of the solid supports, a mixture containing 25 to 150 g of dried precursor powder, prepared as described in Example 1, and 60 to 300 g of isobutyl alcohol is poured into the reservoir of a paint gun and sprayed on the surface of solid supports. The coated catalysts obtained in such a way are then calcined in a vertical tubular furnace as described herein. The carried and catalyst characteristics are detailed in Table 1 as referred to catalysts 5a, 5b and 5c.

### Example 6

This example illustrates the preferred coating procedure of this invention wherein isobutyl alcohol and a wet cake of the precursor are used for the preparation of the slurry.
Approximately 200 ml of solid inert supports are placed in a rotating (20 r.p.m.) cylindrical container and heated at about 50°C by means of a stream of hot air. Depending on the quantity of active material to be deposited on the surface of the solid supports, a mixture of 60 to 300 g of a wet precursor cake, prepared as described in Example 1, and of 60 to 300 g of isobutyl alcohol poured into the reservoir of a paint gun and sprayed on the surface of solid supports. The coated catalysts obtained in such a way are then calcined in a vertical tubular furnace as described herein. The carrier and catalyst characteristics are detailed in Table 1 as referred to catalysts 6a, 6b, 6c, 6d, 6e, 6f and 6g.

### Example 7

This example illustrates the preferred coating procedure of this invention where an organic liquid and a wet cake of the precursor are used for the preparation of the slurry.
Approximately 200 ml of solid inert supports are placed in a rotating (20 r.p.m.) cylindrical container and heated at about 50°C by means of a stream of hot air. Depending on the quantity of active material to be deposited on the surface of the solid supports, a mixture of 60 to 300 g of a wet precursor cake, prepared as described in Example 1, and of 60 to 300g of an organic liquid is poured into the reservoir of a paint gun and sprayed on the surface of solid supports. The coated catalysts obtained in such a way are then calcined in a vertical tubular furnace as described herein. The carrier and catalyst characteristics are detailed in Table 1 as referred to catalysts 7a, 7b, 7c, 7d, and 7e.

### Example 8 (Comparative)

This example is illustrative of the current 100% catalyst technology for making maleic anhydride from aliphatic hydrocarbons and thus is comparative to the instant invention.

Approximately 100 g of catalyst precursor powder, obtained as described in Example 1, are pressed into 5x5 mm cylindrical tablets with a crush strength of 2 kg force as measured by a Stokes hand-held tester. After drying and calcination in a vertical tubular furnace in the same manner as the catalyst of the instant invention, approximately 60 ml of the 100% catalyst tablets are tested in the single tube reactor. The characteristics and performance of the catalyst are detailed in Table 1 as catalyst 8.

The Table 1 reports the characteristics of the carrier, binder, as well as catalyst, the test results of the instant invention and the comparative technology. The active material relates to the coating containing the vanadium phophorus oxides catalyst materials. The coating thickness of the catalysts is measured by first cutting in half the catalyst particles and then making measurements with a Vernier caliper. As a confirming procedure, measurements with a microscope are carried out. The 'Active Mat. per Volume' column relates to the weight of the active material coating the carrier in a unit of bulk volume of the combined carrier and active material, expressed in grams active material per cubic centimeter bulk volume. The Productivity column is the weight/weight ratio of maleic anhydride produced per hour to the weight of active material coated onto the carrier, expressed as grams maleic anhydride per hour per kilogram of active material. Other headings are self explanatory or are described therein.

Table 1 shows that the performance of the instant invention is remarkable, especially bearing in mind that a maximum of less than 30% of active material in the invention catalyst is present in the test reactor. The productivities are very high and generally over three tines that of the 100% catalyst. The demonstrated yields of the catalysts of the instant invention are generally comparable to those of 100% catalysts, however 2e, 2f,6b, 6c, 6d, 6e, 6f, and 6g give higher yield and selectivity. The high selectivities suggest that the coated catalysts can be effectively used in the well known "hot spot" zone in the commercial maleic reactor. Moreover, the catalysts 6b to 6g and 7a to 7e obtained by the coating procedure where an organic solvent and a precursor cake are used for the preparation of the slurry, exhibits higher yields at low reaction temperature than those made in presence of water. Another observation may be made that even with the large size catalyst particles, e.g. 2c and 4e, the performance is good. This envisages lower pressure drops in the long commercial reactor charges, a definite operational and energy saving advantage.

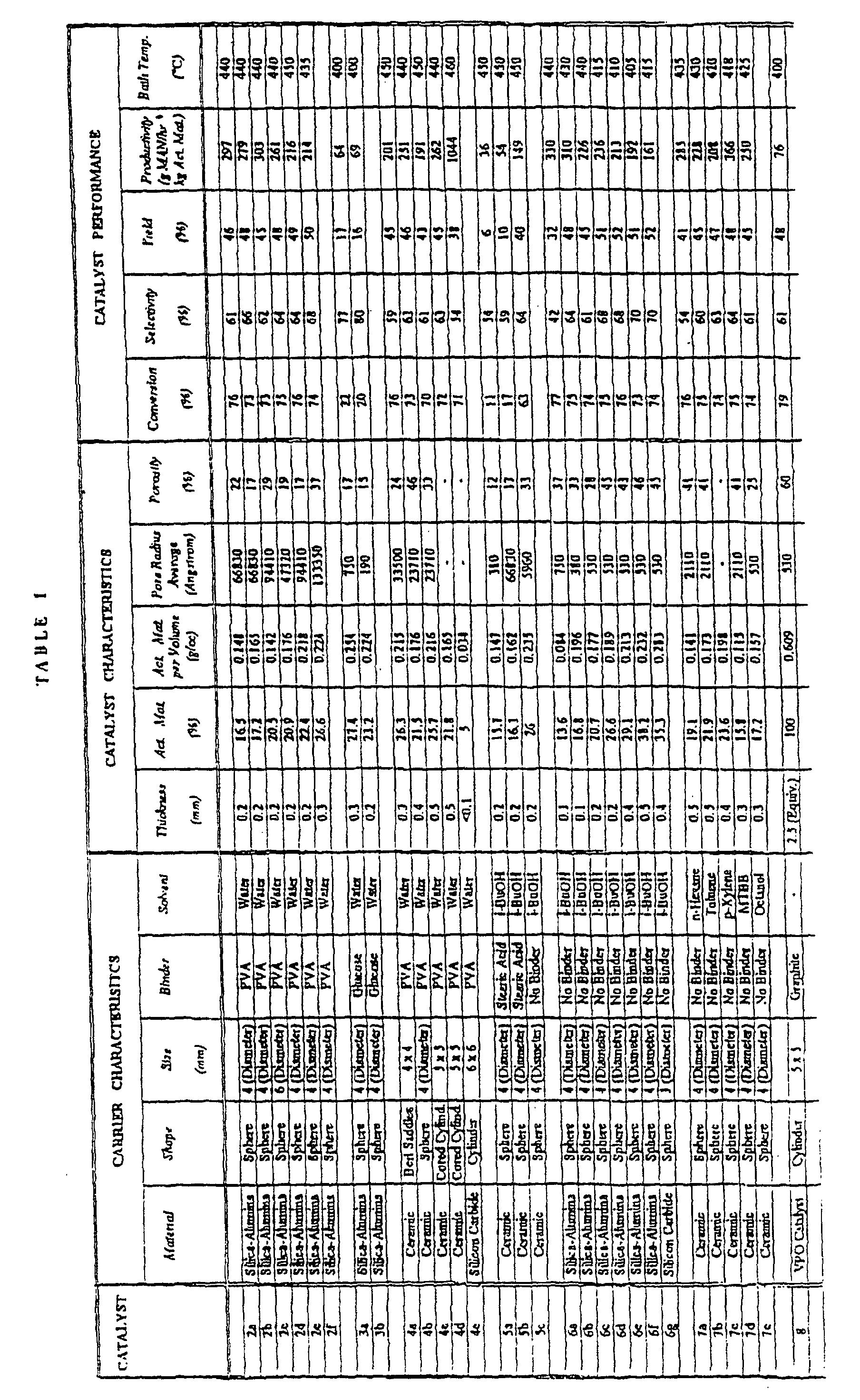

## Claims

1. A process for the preparation of an oxidation catalyst containing an active catalytic material coated in a thin layer on the surface of a solid support, said active catalytic material containing vanadyl pyrophosphate, having an average vanadium valence from 3.8 to 4.5 and a phosphorus to vanadium atomic ratio from 0.8 to 1.5, being present in an amount from 0.02 grams to 0.40 grams per cubic centimeter bulk volume of the combined support and active catalytic material, and having a thickness of from about 0.1 mm to 0.8 mm, which comprises the following steps:
a) providing an active catalyst precursor containing vanadyl hydrogen phosphate hemihydrate;
b) mixing said active catalyst precursor with an organic liquid;
c) coating said mixture onto a solid support;
d) calcining the above mixture coated on the solid support in the presence of a gas mixture of 50/50 air and steam to obtain said active catalytic material, and thus to produce a catalyst with an active catalytic material having a pore radius average of 27 to 250 nm and a percent porosity of at least 30 % and exhibiting a weight/weight productivity to maleic anhydride of at least 200g maleic anhydride/hour per kg active catalytic material based on a performance test conducted in a fixed-bed reactor at a concentration of 1.5 mole percent hydrocarbon in molecular oxygen containing gas, a space velocity of 1400 hr⁻¹ under a pressure of 1 atmosphere absolute, and a temperature sufficient to maintain the hydrocarbon conversion of 60 mole % to 85 mole %.

2. A process as claimed in claim 1 wherein before starting the calcination process, a 50/50 mixture of air and nitrogen is passed over the catalyst.

3. A process as claimed in any one of claims 1 to 2 wherein the organic liquid is selected from the group consisting of alcohols, ethers, ketones, amines, carboxylic acid derivatives and hydrocarbons, or mixtures thereof.

4. A process as claimed in claim 3 wherein the organic liquid contains isobutanol and/or xylene.

5. A process as claimed in any one of claims 1 to 4 wherein said mixture of the catalyst precursor with an organic liquid contains a binder.

6. A process as claimed in claim 5 wherein the binder is a polyol, a sugar or a derivative thereof.

7. A process as claimed in claim 6 wherein said binder is polyethylene glycol or glucose.

8. A process as claimed in any one of claims 1 to 7 wherein said solid support is selected from the group consisting of silica, alumina, silica-alumina, natural and synthetic silicates, silicon carbide, stainless steel, titania, ceramic material and zirconia.

9. A process as claimed in claim 8 wherein said solid support is selected from the group consisting of ceramic, silica-alumina and silicon carbide.

10. A process as claimed in any one of claims 1 to 9 wherein the solid support has a full spherical and cylindrical form with holes and/or indentations on the outer surface, is a cored cylinder, a Berl saddle, or a fragmented particle.

11. A process as claimed in any one of claims 1 to 10 wherein the active catalytic material is calcined in the oxidation reactor.

12. A process as claimed in any one of claims 1 to 10 wherein the active catalytic material is calcined extemal to the oxidation reactor.

13. A process as claimed in any one of claims 1 to 12 wherein the average vanadium valence of the active catalytic material is from 3.9 to 4.3.

14. A process as claimed in any one of claims 1 to 13 wherein the phosphorus to vanadium atomic ratio of the active catalytic material is from 0.9 to 1.2.

15. A process as claimed in any one of claims 1 to 14 wherein the active catalytic material is present in an amount from 0.03 grams to 0.35 grams per cubic centimeter bulk volume of the combined support and active catalytic material.

16. A process as claimed in any one of claims 1 to 15 wherein the percent porosity of the active catalytic material is from 30 to 70 %.

17. A process as claimed in any one of claims 1 to 16 wherein said active catalytic material has an average pore radius within the range of 35 and 220 nm.

18. A process for the production of maleic anhydride in a fixed-bed reactor by oxidation of C₄ to C₁₀ aliphatic hydrocarbons or a mixture thereof with molecular oxygen or a molecular oxygen-containing gas in the vapour phase in the presence of an oxidation catalyst, wherein the catalyst has been prepared by a process as claimed in any one of claims 1 to 17.

19. A phosphorus vanadium mixed oxide catalyst obtainable by the process of any one of claims 1 to 17.

## Patentansprüche

1. Verfahren zur Herstellung eines Oxidationskatalysators, der in einer dünnen Schicht auf der Oberfläche eines festen Trägers ein aktives katalytisches Material enthält, wobei das aktive katalytische Material Vanadyl-Pyrophosphat enthält, das eine mittlere Vanadium-Valenz von 3,8 bis 4,5 und ein Atomverhältnis Phosphor zu Vanadium von 0,8 bis 1,5 aufweist innerhalb eines Betrages von 0,02 g bis 0,4 g pro cm³ Schüttvolumen des kombinierten Trägers und aktiven Katalysatormateriales und das eine Dicke von ungefähr 0,1 mm bis 0,8 mm aufweist, wobei das Verfahren folgende Schritte umfaßt:
a) Herstellen eines aktiven Katalysatorvormateriales enthaltend Vanadyl-Hydrogen-Phosphat-Halbhydrat;
b) Mischen des genannten aktiven Katalysatorvormateriales mit einer organischen Flüssigkeit;
c) Aufziehen der genannten Mischung auf einen festen Träger;
d) Kalzinieren der obigen Mischung aufgezogen auf dem festen Träger in der Anwesenheit einer Gasmischung von 50/50 Luft und Dampf, um das genannte aktive katalytische Material zu erhalten und auf diese Art und Weise einen Katalysator herzustellen mit einem aktiven katalytischen Material, welches einen mittleren Porenradius von 27 bis 250 µm und ein Porösitätsverhältnis von wenigstens 30 % aufweist und das eine Gewichts-/ Gewichtsproduktivität gegenüber Maleinsäureanhydrid zeigt von wenigstens 200 g Maleinanhydrid/Stunde pro kg aktiven Katalysatormateriales basierend auf einem Performance-Test ausgeführt in einem Festbettreaktor bei einer Konzentration von 1,5 Mol.-% Kohlenwasserstoff in molekularen Sauerstoff enthaltenden Gas bei einer Raumgeschwindigkeit von 1.400 hr⁻¹ und einem Druck von 1 Atmosphäre absolut und bei einer ausreichenden Temperatur, um die Kohlenwasserstoffkonversion von 60 Mol.-% bis 85 Mol.-% zu erreichen.

2. Verfahren nach Anspruch 1, bei dem vor dem Beginn des Kalzinierungsprozesses eine 50/50 Mischung von Luft und Stickstoff über den Katalysator geleitet wird.

3. Verfahren nach einem der Ansprüche 1 oder 2, bei dem die organische Flüssigkeit aus der Gruppe bestehend aus Alkoholen, Ethern, Ketonen, Aminen, Carbonsäure-Derivaten oder Kohlenwasserstoffen oder Mischungen daraus ausgewählt wird.

4. Verfahren nach Anspruch 3, bei dem die organische Flüssigkeit Isobutanol und/oder Xylone enthält.

5. Verfahren nach einem der Ansprüche 1 bis 4, bei dem die Mischung des Vorkatalysators mit einer organischen Flüssigkeit ein Bindemittel enthält.

6. Verfahren nach Anspruch 5, bei dem das Bindemittel ein Polyol oder Zucker oder ein Derivat davon ist.

7. Verfahren nach Anspruch 6, bei dem das Bindemittel ein Polyethylenglycol oder Glukose ist.

8. Verfahren nach einem der Ansprüche 1 bis 7, bei dem der genannte feste Träger aus der Gruppe, die besteht aus Silicium, Aluminium, Silica-Aluminium, natur- oder synthetische Silicate, Silikoncarbit, rostfreiem Stahl, Titan, Keramikmaterial und Zirkonia ausgewählt wird.

9. Verfahren nach Anspruch 8, bei dem der feste Träger aus der Gruppe, die aus Keramik, Silica-Aluminium oder Silikoncarbit besteht, ausgewählt wird.

10. Verfahren nach einem der Ansprüche 1 bis 9, bei dem der feste Träger eine vollständig sphärische und zylindrische Form aufweist mit Löchern und/oder Einbuchtungen an der äuβeren Oberfläche und ein Hohlzylinder, ein Berl-Sattel oder ein bruchstückartiges Teil ist.

11. Verfahren nach einem der Ansprüche 1 bis 10, bei dem das aktive katalytische Material in dem Oxidationsreaktor kalziniert wird.

12. Verfahren nach einem der Ansprüche 1 bis 10,
**dadurch gekennzeichnet,**
**daß** das aktive Material extern zum Oxidationsreaktor kalziniert wird.

13. Verfahren nach einem der Ansprüche 1 bis 12, bei dem die durchschnittliche Vanadium-Valenz des aktiven katalytischen Materiales 3,9 bis 4,3 beträgt.

14. Verfahren nach einem der Ansprüche 1 bis 13, bei dem die Phosphor- zu Vanadiumatomrate des aktiven Katalysatormateriales 0,9 bis 1,2 beträgt.

15. Verfahren nach einem der Ansprüche 1 bis 14, bei dem das aktive katalytische Material in einem Betrag von 0,03 gr bis 0,35 gr pro cm³ Massevolumen des kombinierten Trägers und aktiven Katalysatormateriales anwesend ist.

16. Verfahren nach einem der Ansprüche 1 bis 15, bei dem die Porösität in Prozent des aktiven Katalysatormateriales von 30 bis 70 % beträgt.

17. Verfahren nach einem der Ansprüche 1 bis 16, bei dem das aktive Katalysatormaterial einen mittleren Porenradius innerhalb einer Bandbreite von 35 bis 220 µm hat.

18. Verfahren zur Herstellung eines Maleinsäureanhydrids in einem Festbettreaktor durch Oxidation von C₄ zu C₁₀ aliphatischem Kohlenwasserstoffs oder einer Mischung davon mit molekularem Sauerstoff oder einem molekularen Sauerstoff enthaltenden Gases in der Dampfphase in Anwesenheit eines oxidierenden Katalysators, bei dem der Katalysator erzeugt wurde durch ein Verfahren, wie es in einem der Ansprüche 1 bis 17 beansprucht ist.

19. Ein Phosphor-Vanadium-Gemisch-Oxidationskatalysator hergestellt durch ein Verfahren nach einem der Ansprüche 1 bis 17.

## Revendications

1. Procédé de préparation d'un catalyseur d'oxydation contenant une matière catalytique active appliquée en une couche mince sur la surface d'un support solide, ladite matière catalytique active contenant du pyrophosphate de vanadyle, ayant une valence de vanadium moyenne de 3,8 à 4,5 et un rapport atomique phosphore à vanadium de 0,8 à 1,5, étant présente en une quantité de 0,02 g à 0,40 g par centimètre cube de volume apparent du support et de la matière catalytique active combinés, et ayant une épaisseur d'environ 0,1 mm à 0,8 mm, qui comprend les étapes suivantes :
a) la formation d'un précurseur de catalyseur actif contenant de l'hémihydrate d'hydrogénophosphate de vanadyle;
b) le mélange dudit précurseur de catalyseur actif avec un liquide organique;
c) l'application dudit mélange sur un support solide;
d) la calcination du mélange précité appliqué sur le support solide en présence d'un mélange de gaz de 50/50 d'air et de vapeur pour obtenir la matière catalytique active précitée, et pour produire ainsi un catalyseur avec une matière catalytique active ayant un rayon de pore moyen de 27 à 250 nm et une porosité en % d'au moins 30% et montrant une productivité en poids/poids en anhydride maléique d'au moins 200 g d'anhydride maléique/heure par kg de matière catalytique active sur la base d'un test de performance réalisé dans un réacteur à lit fixe à une concentration de 1,5 mole % d'hydrocarbure dans un gaz contenant de l'oxygène moléculaire, une vitesse spatiale de 1400 h⁻¹ sous une pression de 1 atmosphère absolue, et à une température suffisante pour maintenir la conversion d'hydrocarbure de 60 moles % à 85 moles %.

2. Procédé suivant la revendication 1, dans lequel avant de commencer le procédé de calcination, on fait passer un mélange 50/50 d'air et d'azote sur le catalyseur.

3. Procédé suivant l'une ou l'autre des revendications 1 et 2, dans lequel le liquide organique est choisi dans le groupe comprenant les alcools, les éthers, les cétones, les amines, les dérivés d'acide carboxylique et les hydrocarbures ou leurs mélanges.

4. Procédé suivant la revendication 3, dans lequel le liquide organique contient de l'isobutanol et/ou du xylène.

5. Procédé suivant l'une quelconque des revendications 1 à 4, dans lequel ledit mélange du précurseur de catalyseur avec un liquide organique contient un liant.

6. Procédé suivant la revendication 5, dans lequel le liant est un polyol, un sucre ou un dérivé de ceux-ci.

7. Procédé suivant la revendication 6, dans lequel ledit liant est du polyéthylène glycol ou du glucose.

8. Procédé suivant l'une quelconque des revendications 1 à 7, dans lequel le support solide précité est choisi dans le groupe comprenant la silice, l'alumine, la silice-alumine, les silicates naturels et synthétiques, le carbure de silicium, l'acier inoxydable, l'oxyde de titane, les matières céramiques et la zircone.

9. Procédé suivant la revendication 8, dans lequel ledit support solide est choisi dans le groupe comprenant les matières céramiques, la silice-alumine et le carbure de silicium.

10. Procédé suivant l'une quelconque des revendications 1 à 9, dans lequel ledit support a une forme sphérique et cylindrique pleine avec des trous et/ou des encoches sur la surface extérieure, est un cylindre creux, une scelle de Berl ou une particule fragmentée.

11. Procédé suivant l'une quelconque des revendications 1 à 10, dans lequel la matière catalytique active est calcinée dans le réacteur d'oxydation.

12. Procédé suivant l'une quelconque des revendications 1 à 10, dans lequel la matière catalytique active est calcinée à l'extérieur du réacteur d'oxydation.

13. Procédé suivant l'une quelconque des revendications 1 à 12, dans lequel la valence de vanadium moyenne de la matière catalytique active est de 3,9 à 4,3.

14. Procédé suivant l'une quelconque des revendications 1 à 13, dans lequel le rapport atomique phosphore à vanadium de la matière catalytique active est de 0,9 à 1,2.

15. Procédé suivant l'une quelconque des revendications 1 à 14, dans lequel la matière catalytique active est présente en une quantité de 0,03 g à 0,35 g par centimètre cube de volume apparent du support et de la matière catalytique active combinés.

16. Procédé suivant l'une quelconque des revendications 1 à 15, dans laquelle la porosité en % de la matière catalytique active est de 30 à 70%.

17. Procédé suivant l'une quelconque des revendications 1 à 16, dans lequel ladite matière catalytique active a un rayon de pore moyen allant de 35 à 220 nm.

18. Procédé de production d'anhydride maléique dans un réacteur à lit fixe par oxydation d'hydrocarbures aliphatiques en C₄ à C₁₀ ou d'un mélange de ceux-ci avec de l'oxygène moléculaire ou un gaz contenant de l'oxygène moléculaire en phase vapeur en présence d'un catalyseur d'oxydation, dans lequel le catalyseur a été préparé par un procédé suivant l'une quelconque des revendications 1 à 17.

19. Catalyseur d'oxyde mixte de phosphore et de vanadium obtenable par le procédé suivant l'une quelconque des revendications 1 à 17.
